# EUROPEAN PATENT APPLICATION

(11) **EP 2 567 712 A1**
(43) Date of publication of application: **13.03.2013**
(21) Application number: 11180415.9
(22) Date of filing: 07.09.2011
(51) Int. Cl.: A61L 2/10, A61L 2/08, A01J 25/16, A23C 19/064, C02F 1/32

(54) **Microbial reduction in liquid**

(71) Applicant: Tetra Laval Holdings & Finance S.A., 1009 Pully (CH)
(72) Inventor: Wuensche, Thomas, 21514 Buechen (DE)
(74) Representative: Tetra Pak - Patent Attorneys SE

(57) **Abstract**

The present invention relates to purification of liquid in a food processing system, such as a beverage or brine from cheese brining. A method, device, system and use for antimicrobial treatment and simultaneous particle reduction, without adding chemicals, are also provided.

## Description

### Technical Field

This invention pertains in general to the field of continuous liquid purification. More particularly the invention relates to continuous microbial reduction in liquid in a food processing system, such as a beverage or brine used in cheese manufacturing.

### Background

In the foodstuff industry, processing of liquids is common. For example, when manufacturing cheese, a step of brining, or salting, is usually included to control the taste and texture of the cheese. In the brining process, salt is absorbed by the cheeses and whey is expelled from it. In time, the whey will thus comprise dissolved solids along with curd particles, microorganisms and fat. If the microorganisms are salt tolerant, they may multiply in the brine, which is highly undesirable.

Previously, a simple replacement of old brine with new brine, or addition of chemicals, provided cleaning of the system. However, with increased demands for low environmental impact it is often necessary to clean and recycle the brine.

US4815368A discloses a system for brining cheese blocks. The brine is recirculated through a filter for removal of solid particles and then undergoes UV-treatment to reduce microorganisms. The use of two separate cleaning units makes this method cumbersome. Furthermore, the filter must be cleaned, which makes it hard to continuously run the brine recycling. Also, sterilizing with UV light is energy consuming.

Another solution may be to apply a nano filter, which is capable of removing both solids and microorganisms. A nano filter is however expensive, and may lead to undesirable pressure drops in the system.

Devices for purifying liquids with AOT are known from other technical fields, such as disclosed in WO 2010/002351 A1.

### Summary

Accordingly, the present invention preferably seeks to alleviate or eliminate one or more of the above-identified deficiencies in the art and disadvantages singly or in any combination and solves at least the above mentioned problems by providing a device, a method and a use according to the appended patent claims.

The general solution according to the invention is a single unit comprising both a photocatalytic oxidant generator with a catalytic surface and a filter, wherein the filter is the catalytic surface and the filter has a mesh size below 50 µm.

In a first aspect, a method for purification of a liquid in a food processing system is provided. The method comprises contacting a filter, said filter comprising a metal and/or a metal oxide, such as Au, Pt, and/or Ti0₂, with a flow of said liquid and irradiating the filter with light creating free radicals, such that the liquid is purified by destroying polluting particles.

This has the advantage over the prior art that it provides antimicrobial treatment and simultaneous particle reduction without adding chemicals, which is energy efficient and easy to operate.

The method may further comprise flushing the first side of the filter with a clean flow, separate from the flow of liquid.

This is advantageous, because it may rinse the surface of the membrane thus providing better access for the light, which improves efficiency.

In an embodiment, the method further comprises sterilizing the liquid in a subsequent sterilizing step.

This is advantageous, because it may increase the purity of the liquid.

The light may be UV light, which is advantageous, because it provides efficient creation of free radicals.

In an embodiment, the liquid is a beverage. The beverage may be drinking water, a dairy product or juice.

In another embodiment, the liquid is brine, such as brine form cheese brining.

In a second aspect, a device for continuous purification of liquid in a food processing system is provided. The device comprises a photo catalytic oxidant generator with a catalytic surface and a filter, wherein the filter comprises the catalytic surface and has a mesh size below 50 µm.

This is advantageous, because it provides good purification of the liquid.

The device may have a catalytic surface which is a metal oxide, such as Ti0₂.

This is advantageous, because it provides a strong photocatalytic creation of free radicals.

In an embodiment, the device comprises UV lights for irradiating the catalytic surface.

This is advantageous, because it provides a strong photocatalytic creation of free radicals.

In an embodiment, the mesh of the filter in the device is between 1 µm and 10 µm.

This provides the advantage that the filter may purify the liquid from bacteria.

In a third aspect, a system for continuous purification of liquid in a food processing system comprising a device according to the second aspect is provided, wherein said device is connected to a storage tank through a conduit.

In an embodiment, the system further comprises a rinsing circuit connected to the device, for rinsing the filter.

This is advantageous, because it may rinse the filter efficiently, which ensures proper creation of free radicals on the surface of the filter. It may also provide a way to collect waste from the system.

In a fourth aspect, a use of the device according to the second aspect, or the system according to the third aspect, for purification of liquid in a food processing system is provided.

The liquid may be a beverage, such as drinking water, a dairy product or juice.

The liquid may also be brine from a cheese brining process.

The present invention has the advantage over the prior art that it provides antimicrobial treatment and simultaneous particle reduction without adding chemicals, which is energy efficient and easy to operate.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic overview of method steps according to an embodiment;
Fig. 2 is process scheme of a system according to an embodiment;
Fig. 3A is a schematic side view of an embodiment and Fig. 3B and 3C are two perspective views of an embodiment;
Fig. 4A is a partial view of a filter according to an embodiment, and Fig. 4B is a partial view of a filter according to another embodiment;
Fig. 5 is a cross sectional side view of a filter according to an embodiment, Fig. 5A depicts operation in normal mode and Fig. 5B depicts operation in flush mode; Fig. 6 is a schematic perspective view of an embodiment;
Fig. 7Ais a schematic side view of an embodiment, Fig. 7B is a schematic bottom view of the same embodiment; and
Figs 8A and 8B are perspective views of an embodiment.

### Description of embodiments

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

The following description focuses on an embodiment of the present invention applicable to a method for purifying brine used for making cheese. Without being bound by theory, it is thought that the surprisingly good purifying effect may be contributed by the salt in brine, which typically has a salt content above 5 %, such as between 6 % and 23 %, stabilizes free radicals which provides a more effective purification.

However, it is submitted that the method may be equally useful for treating any liquid in a food processing system, such as any kind of food liquid, such as beverage or soup. Examples of such beverage are drinking water, juice, milk, yoghurt, etc., and examples of soup are tomato soup, potato soup, mushroom soup, etc.

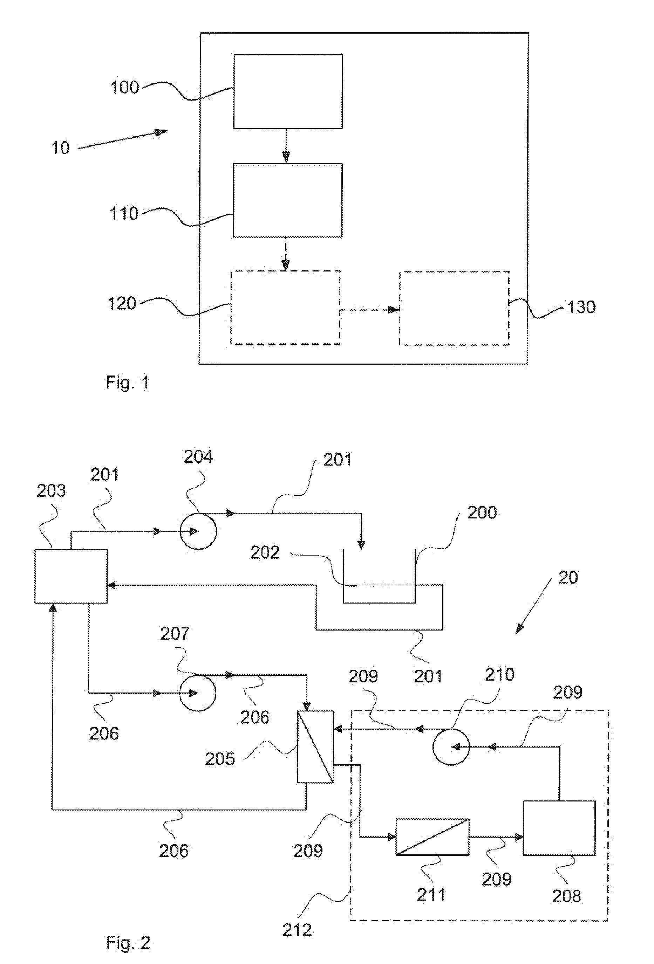

In an embodiment according to Fig. 1, a method 10 for continuous purification of brine is provided. The method 10 comprises contacting 100 a filter comprising a catalyst surface in form of a metal oxide surface, such as TiO₂, Au, or Pt, with a flow of brine to be purified, such as brine from a cheese brining process. The method further comprises irradiating the filter 110 with light, such as light comprising a UV light component, creating free radicals, which purify the brine by destroying polluting particles.

It is surprising that such good purification effect is obtained in a solution, such as brine, comprising a relatively high amount of denaturated proteins, since it would be expected that the catalyzing effect would drop severely when the denaturated proteins stick to the catalyst surface. This has however not been the case, even though it is preferred to clean the catalyst surface at intervals. In the same way, it is surprising that food liquids with a protein content above 0.005 %(by weight), such as brine, or even above 1 % (by weight), such as low fat dairy products, fruit juices, etc., or even a protein content between 2 and 20 % (by weight), such as milk, etc., or even a protein content between 3 and 15 % (by weight), such as high fat milk, cream, may be advantageously treated by the method according to the present invention. In this context protein content is to be interpreted as the total protein content, including both denaturated and non-denaturated proteins.

The filter is positioned so that unfiltered brine contacts a first side, penetrates the filter and exits on a second side of the filter as filtered brine. Irradiating the filter 110 may be performed on the first side of the filter, on the second side, or on both.

In an embodiment, the method further comprises flushing 120 the first side of the filter with a clean flow, which is unpolluted and separate from the flow of brine.

This is advantageous, since proteins and other catalyst surface adhering substances may be removed from the catalyst surface, thus providing less clogging of the filter, and consequently lowering the pressure drop over the filter unit.

In an embodiment, the method further comprises sterilizing 130 the liquid in a subsequent sterilizing step. This is advantageous, since it provides additional purification of the liquid, which may be called for with regard to certain food products.

### System configuration

In an embodiment according to Fig. 2, a system 20 for continuous purification of brine is provided. However, it is submitted that other systems, suitable for processing other liquid in a food processing system, such as any kind of food liquid, such as beverage or soup, are equally possible, and these systems may naturally not be limited to the system specified here - specifically not in relation to cheese and brine specific features, such as cheese holders. The system 20 comprises a brining tank 200, which comprises cheese (not shown) undergoing a brining process, and brine. The brine is allowed to circulate from and to the brining tank through a first set of pipes 201. The brining tank 200 comprises holders 202 adapted to hold cheese. The holders may be direct holders, such as hooks or fixed cheese racks or sieves, or indirect holders, such as flanges or lips at the inner surface of the tank for holding a cheese rack or sieve. In this way the cheese may be positioned such that the brine in the brining tank 200 may access all sides of the cheese. This is advantageous, since it provides more effective brining.

The brine may be stored in a storage tank 203 and be circulated by means of a first pump 204, arranged along the first set of pipes 201.

The cheese will pollute the brine with proteins and particles. The content of particles and protein may be above 0.05 % (by weight). The content of protein may be above 0.005 % (by weight). These may provide good conditions for microorganisms to grow.

Thus, the system comprises a device 205 for continuous purification of liquid in a food processing system, such as an AOT unit (described below). The brine circulates in a conduit through a second set of pipes 206, by means of a second pump 207. By means of the device 205 for continuous purification of liquid in a food processing system, the brine is purified. Waste, which is filtered during the purification, may be removed from the device 205 for continuous purification of liquid in a food processing system by means of a rinsing fluid, stored in a rinsing fluid tank 208. The rinsing fluid is circulated through a third set of pipes 209 by means of a third pump 210, thus forming a rinsing circuit 213. An advantage with this is that the device 205 may be cleaned in a continuous fashion, whenever needed. The waste may be filtered from the rinsing fluid by means of a filter 211 and discarded. The rinsing fluid may be recirculated.

The system may provide better brine quality, with fewer by-products and less maintenance. Thus, the system is safe and cheap. The system also use high flow rates, such as up to 45 m³/h per system, such as 20 to 25 m³/h. At these high flow rates a content of protein and particles above 0.05 % (by weight) may severely hamper the purification effect of the AOT unit.

Also, since no additional chemicals are required, the system is safe to the environment.

### Advanced oxidation treatment

Advanced Oxidation Technology (AOT) is a collective term for several technologies designed to effectively remove harmful bacteria and other chemical and biological substances in water. Common for all of these is that they have a low environmental impact.

The technology is based on TiO₂, which, when it is subjected to radiation exceeding its band gap, generates electron-hole pairs so that additional electrons enter the conduction band, while holes remain in the valence band. These photo-generated electron-hole pairs facilitate redox reactions creating free radicals, such as superoxide anions or hydroxyl along the Ti0₂ surface. Examples of such redox reactions are

*O*₂ → ● *O*⁻₂ + *e*⁻;

or

*H*₂*O* → ● *OH* + *H*⁺.

The photocatalytic activity of TiO₂ depends on the relative rates of generation and recombination of electron-hole pairs as well as the levels of radical-forming species along the Ti0₂ surfaces.

Thus, by irradiating Ti0₂ with light, especially UV light, free radicals are generated. The free radicals hit the surface of microorganisms and other contaminants and lead to a degradation of the contaminant. This is advantageous, since it provides effective purification.

When the radical reacts with the contaminant, it is turned back to its original state, such as water. This is advantageous, because of low environmental impact. Operation of AOT does not demand addition of chemicals or create harmful residues.

Also, UV light needed to create radicals with a degrading effect on microorganisms is much less compared to if the UV light would be used to degrade microorganisms to the same degree. Thus, use of AOT saves energy.

### AOT Unit

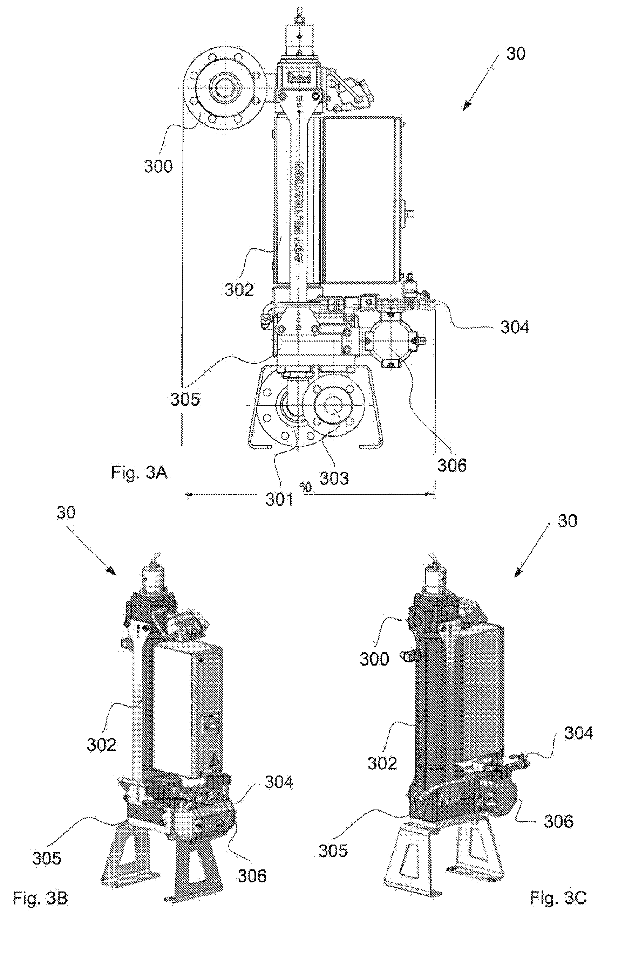

In an embodiment according to Fig. 3, an advanced oxidation treatment (AOT) unit 30 is provided. The unit 30 comprises an inlet 300 and an outlet 301, for connection to the second set of pipes 206 described above, and circulate brine (not shown). The AOT unit 30 also comprises a filter chamber 302, which is located between the inlet 300 and the outlet 301 so that brine can pass through it.

Fig. 3Ais a schematic side view of the AOT unit 30 and Fig. 3B and 3C shows two perspective views of the AOT unit.

The AOT unit further comprises a waste outlet 303 for directing waste, which is filtered during the purification, from the stream of brine. Rinsing fluid (not shown) may be added to the filter chamber 302 by rinsing inlets 304.

This is advantageous, because it allows effective, automatic flushing of the filter chamber 302.

The unit also comprises flushing regulators, such as pumps, for dispensing the rinsing fluid. This is advantageous, since it provides an opportunity to optimize the flushing operation by dispensing the fluid in pulses, or dispense a mixture of rinsing liquid and air, or a continuous, intensive flush stream.

The waste outlet 303 and the rinsing inlets 304 may be connected to the third set of pipes 209 described above.

The AOT unit may further comprise a settling unit 305, downstream of the filter chamber 302, to allow settling of particles in either the stream of brine or the stream of rinsing fluid.

Also, the AOT unit 30 may comprise an outflow regulator 306, which directs the flow into the outlet 301, the waste outlet 303, or both.

The AOT unit is advantageous, because it is self cleaning. It also has few moving parts and few consumables.

### Filter

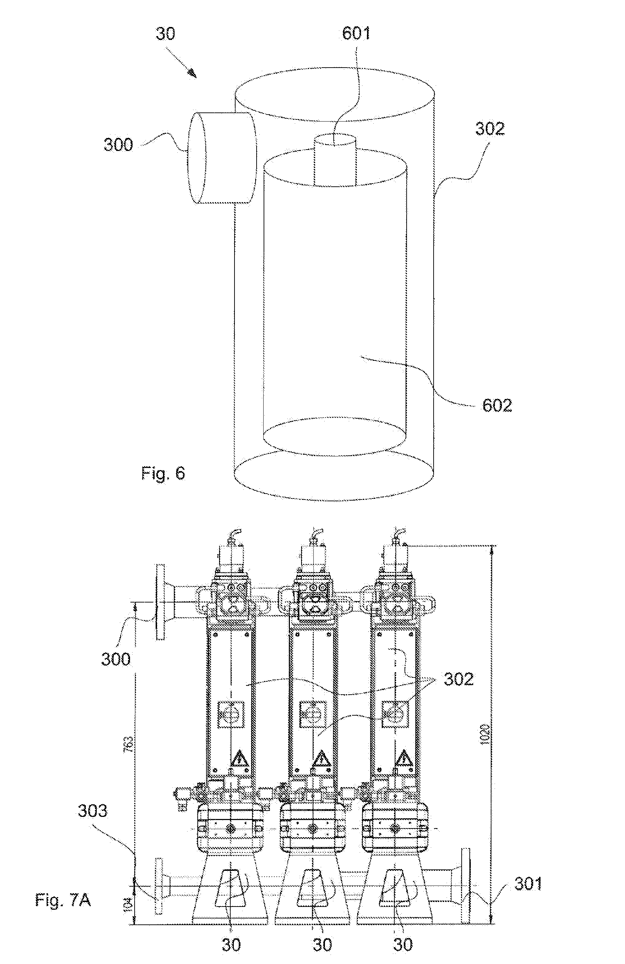

The AOT unit comprises a filter and a light source, such as UV lights. In an embodiment according to Fig. 6, UV lights 601 have been placed next to a filter 602, on the first side of the filter 602, i.e. the side first being contacted with the brine. Both the UV lights 601 and the filter 602 are placed inside the filter chamber 302.

The filter may comprise a carrier structure, such as a metal grid, whereupon metal or metal oxide, such as Ti0₂, Au, Pt, etc., is coated. The metal grid may be stainless steel. One way of coating the carrier structure is by sintering Ti0₂ onto the surface of the carrier, which will be appreciated by a person skilled in the art.

In an embodiment, the metal oxide, such as Ti0₂, is doped, such as with nitrogen (N), carbon (C), and/or boron (B). Due to the relatively lower electronegativity, C and B atom can intensively affect Ti0₂ charge distribution. Since, only ultraviolet radiation could be absorbed by the TiO₂ alone, and the presence of protein coatings etc. on the catalyst surface could limit the amount of ultraviolet radiation reaching the catalyst surface, doping with N, C, and/or B increase absorption of energy. Without being bound to theory, this could be achieved by the low or no mixing of the C or B atom 2p bands with 0 2p bands, because the matching of p bands decreases along with the difference in electronegativity. Also, doping may improve capability to create free radicals under influence of light.

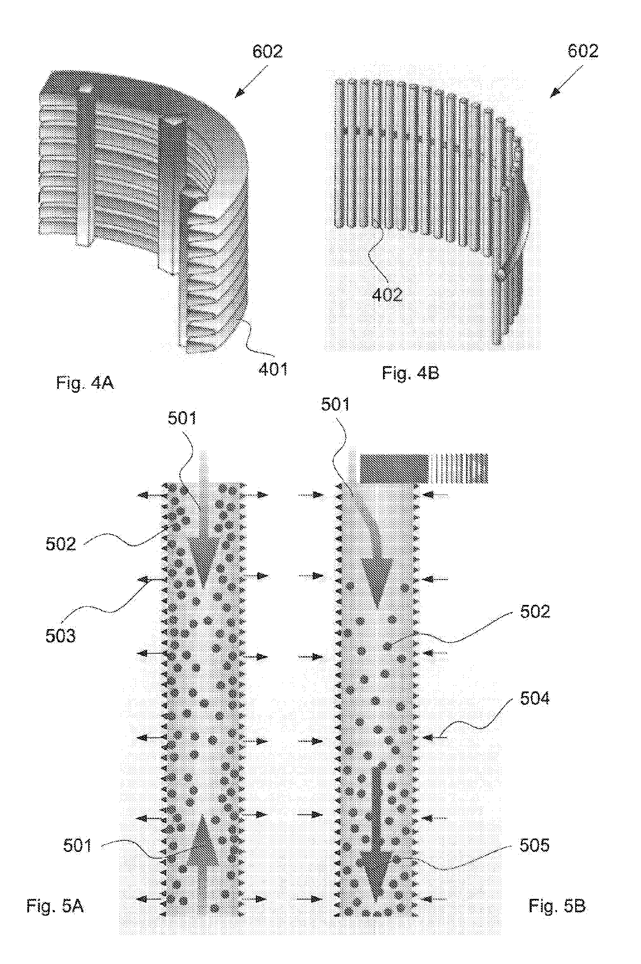

Fig. 4 provides examples of filter 602. Fig. 4A provides a filter structure where filter lamellas 401 are positioned as rings, parallel to each other. The rings are positioned so that the radius of the ring coincides with the radius of the filter chamber 302. Fig. 4B provides a filter structure where filter rods 402 are positioned parallel to each other along the length of the filter chamber 302, i.e. substantially perpendicular to the radius of the filter chamber 302.

The mesh size of the filter 602 may be below 50 µm, such as between 0.5 µm to 45 µm, such as between 1 µm and 10 µm. This is advantageous, since a small mesh size gives efficient cleaning. Typically, bacteria have a size of between 6 µm and 12 µm. Thus, if the filter has a mesh size smaller than 10 µm, such as between 1 µm and 10 µm, it has the capacity to purify the liquid from bacteria. It is surprising that a filter with such small mesh size would work in an environment where proteins or salt might clog the membrane. It might be expected that proteins from a liquid with high protein content would form aggregates, or that salt from brine (with a salt content typically between 6 % and 23 %) would crystallize, and thus clog the filter.

In use, the filter may be cleaned by a stream of rinsing liquid, as described above, such as via automatic back flushing. Automatic back flushing requires a system air pressure of 6-7 bar.

This is advantageous; since a filter with a mesh size below 50 µm may then be used even to filter liquids with high protein content or high salt content.

Fig. 5A is a cross sectional side view of a filter operating in normal mode. A stream 501 of brine with polluting particles 502 enters the filter. When the polluting particles 502 engage the first surface of the filter, their passage is hindered by the filter mesh. Thus, a stream 503 of clean brine exits through the filter.

When needed, the filter may be flushed by adding a stream 504 of rinsing liquid. The polluting particles 502 may then be flushed from the filter via an exit flow 505. Fig. 5B is a cross sectional side view of a filter operating in flush mode.

The AOT unit may comprise pressure sensors (not shown) on each side of the membrane. This is advantageous, because a pressure drop can thus be detected, which may indicate that flushing is needed. Flushing may thus be triggered by either pressure drop over the filter, manual operation, time schedule or a combination.

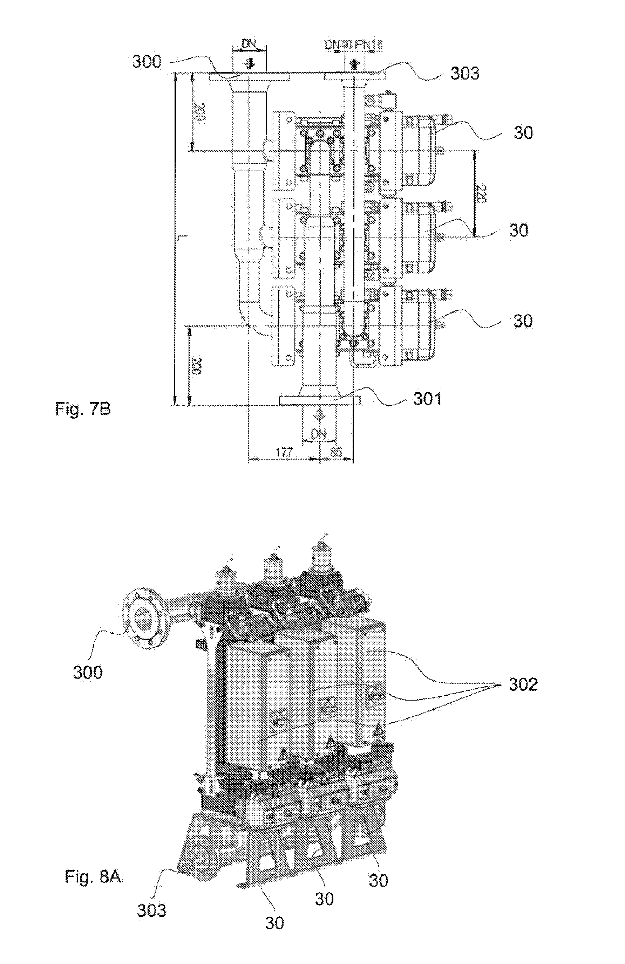

In an embodiment according to Fig. 7, several AOT units 30 are connected in series, such as three AOT units. Fig. 7A is a side view and Fig. 7B is a bottom view.

This is advantageous, since it provides improved cleaning capability.

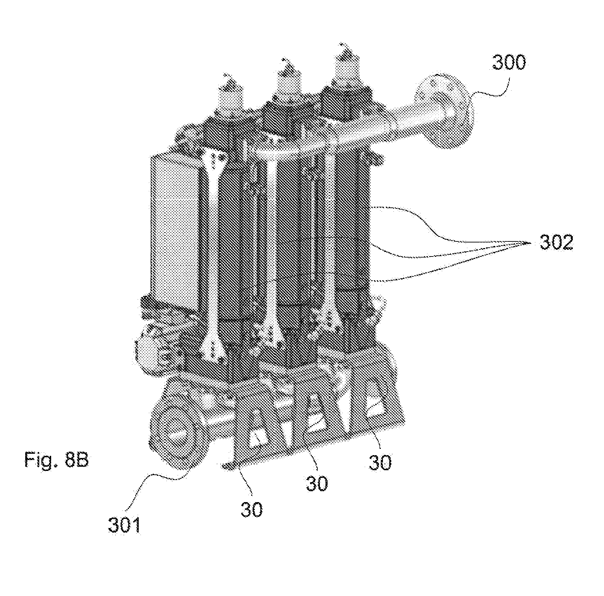

Fig. 8A and 8B are two perspective views of series of AOT units 30.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A method (10) for purification of a liquid in a food processing system, comprising contacting (100) a filter (602), said filter (602) comprising Ti0₂, with a flow of said liquid and irradiating the filter (110) with light creating free radicals, such that the liquid is purified by destroying polluting particles.

2. The method (10) according to claim 1, further comprising flushing (120) the first side of the filter with a clean flow, separate from the flow of liquid.

3. The method (10) according to claims 1 or 2, further comprising sterilizing (130) the liquid in a subsequent sterilizing step.

4. The method (10) according to any of the preceding claims, wherein the light is UV light.

5. The method (10) according to any of the preceding claims, wherein the liquid is a beverage.

6. The method (10) according to claim 5, wherein the beverage is drinking water, a dairy product or juice.

7. The method (10) according to any of claims 1 to 4, wherein the liquid has a salt content above 5 % (by weight).

8. A device (205, 30) for continuous purification of liquid in a food processing system, comprising a photo catalytic oxidant generator with a catalytic surface and a filter (602), wherein the filter (602) comprises the catalytic surface and has a mesh size below 50 µm.

9. The device (205, 30) according to claim 8, wherein the catalytic surface is a metal or metal oxide, such as Au, Pt, or Ti0₂.

10. The device (205, 30) according to any of claims 8 or 9, comprising energy emitting units, capable of emitting UV light (601), for irradiating the catalytic surface.

11. The device (205, 30) according to any of claims 8 to 10, wherein the mesh of the filter (602) is between 1 µm and 10 µm.

12. A system (20) for continuous purification of liquid in a food processing system comprising a device (205) according to any of claims 8 to 11, wherein said device (205) is connected to a storage tank (203) through a conduit (206).

13. The system (20) according to claim 12, further comprising a rinsing circuit (212) connected to the device (205), for rinsing the filter (602).

14. Use of the device according to any of claims 8 to 11, or the system according to any of claims 12 to 13, for purification of liquid in a food processing system.

15. Use according to claim 14, wherein the liquid is a beverage.

16. Use according to claim 15, wherein the beverage is drinking water, a dairy product or juice.

17. Use according to claim 14, where the liquid is brine from a cheese brining process.

## Amended claims

### Amended claims in accordance with Rule 137(2) EPC.

**1.** A method (10) for purification of a liquid in a food processing system, said liquid having a protein content above 0,005 % (by weight), **characterized by**:
contacting (100) a filter (602), said filter (602) comprising a catalytic surface of a metal or metal oxide, with a flow of said liquid; and irradiating the filter (110) with light creating free radicals, such that the liquid is purified by destroying polluting particles.

**2.** The method (10) according to claim 1, further comprising flushing (120) the first side of the filter with a clean flow, separate from the flow of liquid.

**3.** The method (10) according to claims 1 or 2, further comprising sterilizing (130) the liquid in a subsequent sterilizing step.

**4.** The method (10) according to any of the preceding claims, wherein the light is UV light.

**5.** The method (10) according to any of the preceding claims, wherein the liquid is a beverage.

**6.** The method (10) according to claim 5, wherein the beverage is drinking water, a dairy product or juice.

**7.** The method (10) according to any of claims 1 to 4, wherein the liquid has a salt content above 5 % (by weight).

**8.** A device (205, 30) for continuous purification of liquid in a food processing system, **characterized by** a photo catalytic oxidant generator with a catalytic surface and a filter (602), wherein the filter (602) comprises the catalytic surface and has a mesh size between 0.5 µm to 45 µm.

**9.** The device (205, 30) according to claim 8, wherein the catalytic surface is a metal or metal oxide, such as Au, Pt, or TiO₂.

**10.** The device (205, 30) according to any of claims 8 or 9, comprising energy emitting units, capable of emitting UV light (601), for irradiating the catalytic surface.

**11.** The device (205, 30) according to any of claims 8 to 10, wherein the mesh of the filter (602) is between 1 µm and 10 µm.

**12.** A system (20) for continuous purification of liquid in a food processing system comprising a device (205) according to any of claims 8 to 11, wherein said device (205) is connected to a storage tank (203) through a conduit (206).

**13.** The system (20) according to claim 12, further comprising a rinsing circuit (212) connected to the device (205), for rinsing the filter (602).

**14.** Use of the device according to any of claims 8 to 11, or the system according to any of claims 12 to 13, for purification of liquid in a food processing system.

**15.** Use according to claim 14, wherein the liquid is a beverage.

**16.** Use according to claim 15, wherein the beverage is drinking water, a dairy product or juice.

**17.** Use according to claim 14, where the liquid is brine from a cheese brining process.
